# EUROPEAN PATENT APPLICATION

(11) **EP 4 473 929 A1**
(43) Date of publication of application: **11.12.2024**
(21) Application number: 24180807.0
(22) Date of filing: 07.06.2024
(51) Int. Cl.: A61B 18/14, A61B 34/20, A61B 90/00, A61B 18/00

(54) **BASKET CATHETER WITH DEFORMATION SENSOR RELYING ON EDDY CURRENT**

(30) Priority: 09.06.2023 US 202363507164 P; 08.05.2024 US 202418658048
(71) Applicant: Biosense Webster (Israel) Ltd., 2066717 Yokneam (IL)
(72) Inventor: SHAMELI, Ehsan, Irvine, 92618 (US); VAN NIEKERK, Pieter Emmelius, Irvine, 92618 (US); EBRAHIMI, Babak, Irvine, 92618 (US)
(74) Representative: Carpmaels & Ransford LLP

(57) **Abstract**

A basket catheter and system is configured to determine a force on a basket assembly of the basket catheter and/or determine deformation of the basket assembly by using a sensor assembly in the basket catheter which includes a transmitting coil and multiple receiving coils in the catheter shaft and an electrically conductive plate, disk, or ring at a distal end of the basket assembly. The system is configured to provide signals to the transmitting coil to generate a first magnetic field that in turn causes eddy currents in the electrically conductive plate, disk, or ring, that in turn generates a second magnetic field. The receiving coils detect a superposition of the first and second magnetic fields, and the system determines the force and/or deformation based at least in part on the signals from the receiving coils.

## Description

### CROSS-REFERENCE TO RELATED APPLICATIONS

This application claims priority to prior filed U.S. Patent Application No. 63/507,164 filed on June 9, 2023, Attorney Docket No. BIO6818USPSP1-253757.000356 which is hereby incorporated by reference as set forth in full herein.

### FIELD

The present invention relates generally to medical devices, and in particular to multi-electrode basket catheters.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Regions of cardiac tissue can be mapped by a catheter to identify the abnormal electrical signals. Electrophysiology (EP) mapping may include the use of sensing electrodes configured to monitor electrical signals within the cardiovascular system to pinpoint the location of aberrant conductive tissue sites that are responsible for the arrhythmia. Examples of an EP mapping system are described in U.S. Patent No. 5,738,096, incorporated herein by reference.

By selectively ablating cardiac tissue by application of energy via a catheter, it is sometimes possible to cease or modify the propagation of unwanted electrical signals from one portion of the heart to another. The same or different catheter can be used to perform ablation. Catheters configured for mapping and/or ablation typically include at least one electrode and/or a multi-electrode end effector coupled to a distal end of a shaft the catheter.

Catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, Calif.

Many contemporaneous ablation approaches utilize radiofrequency (RF) electrical energy to heat tissue. Cryoablation and irreversible electroporation (IRE) are alternative ablation techniques. Cryoablation is a process that uses extreme cold to destroy tissue. Both RF and IRE utilize electrical signals applied to tissue. However, IRE is considered non-thermal because IRE does not rely on heat to damage tissue; instead, electrical signals applied during IRE result in an unrecoverable permeabilization of cell membranes.

A given catheter for an AF procedure can be configured for mapping, for ablation, or both. The distal portion of a catheter is configured to traverse vascular to be positioned in a heart and/or adjacent vessels. With the exception of catheters configured exclusively for cryoablation, the distal portion of the catheter includes one or more electrodes configured to monitor electrical signals within the cardiovascular system and/or configured for ablation by RF and/or IRE. The distal portion of the catheter can have a variety of configurations. A basket catheter is one such variety having spines that extend along a longitudinal axis when collapsed for delivery through vasculature and that expand away from the longitudinal axis to form a basket shape when deployed at a target site. The spines carry electrodes for sensing and/or ablation.

### SUMMARY

Examples presented herein generally include a basket catheter and an IGS system configured to determine a force on a basket assembly of the basket catheter and/or determine deformation of the basket assembly by using a sensor assembly in the basket catheter which includes a transmitting coil and multiple receiving coils in the catheter shaft and an electrically conductive plate, disk, or ring at a distal end of the basket assembly. The IGS system is configured to provide signals to the transmitting coil to generate a first magnetic field that in turn causes eddy currents in the electrically conductive plate, disk, or ring, that in turn generates a second magnetic field. The receiving coils detect a superposition of the first and second magnetic fields and send signals to the IGS system which determines the force and/or deformation based at least in part on the signals from the receiving coils.

An exemplary medical probe includes a shaft, spines, electrodes, a transmitting coil, a location element, and receiving coils. The shaft extends along a longitudinal axis. The spines extend from a distal end of the shaft and are configured to expand away from the longitudinal axis to form a resilient basket. The electrodes are coupled to each spine. The transmitting coil is disposed within the shaft, aligned along the longitudinal axis and configured to generate a first alternating magnetic field. The location element is coupled to distal ends of the spines. The location element is configured to move in relation to the transmitting coil in response to a force on the resilient basket. The location element is configured to generate a second alternating magnetic field based at least in part on the first alternating magnetic field and based in part on a position of the location element in relation to the transmitting coil. The receiving coils are disposed within the shaft and are each configured to output a respective electrical signal based at least in part on a superposition of the first alternating magnetic field and the second alternating magnetic field.

An exemplary medical system includes a signal generator and a signal processor. The signal generator can be configured to provide an output signal to a transmitting coil disposed in a shaft of a medical probe. The signal processor can be configured to receive a input signals from receiving coils disposed in the shaft of a multi-electrode basket catheter and determine a position of an electrically conductive, non-ferromagnetic element coupled to distal ends of spines of the multi-electrode basket catheter based at least in part on a comparison of the output signal to the input signals.

An exemplary method can include one or more of the following steps performed in various orders and with interleaving steps as understood by a person skilled in the art. The method can include generating a first alternating magnetic field from a transmitting coil disposed in a shaft of a multi-electrode basket catheter; inducing eddy currents on an electrically conductive, non-ferromagnetic element coupled to distal spine ends of a basket assembly of the multi-electrode basket catheter; inducing respective electrical signals in receiving coils disposed in the shaft of the multi-electrode basket catheter such that the respective electrical signals are based at least in part on a superposition of the first alternating magnetic field and a second alternating magnetic field generated by the eddy currents; and determining a position of the electrically conductive, non-ferromagnetic element based at least in part on the respective electrical signals.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements. The figures depict one or more implementations of the inventive devices, by way of example only, not by way of limitation.
Figure 1 is an illustration of an exemplary catheter-based electrophysiology mapping and ablation system according to aspects of the present invention.
Figure 2 is an illustration of a distal portion of an exemplary catheter according to aspects of the present invention.
Figure 3 is an illustration of a coil assembly and locating element of the catheter according to aspects of the present invention.
Figure 4 is an illustration of a transmitting coil inducing a first alternating magnetic field, which induces eddy currents on a location element, thereby inducing a second alternating magnetic field according to aspects of the present invention.
Figure 5A is an illustration of deformation of a basket assembly of the catheter in response to a force in an x-direction according to aspects of the present invention.
Figure 5B is an illustration of the location element and receiving coils of the catheter of Figure 5A according to aspects of the present invention.
Figure 6 is an illustration of deformation of the basket assembly of the catheter in response to a force in a z-direction according to aspects of the present invention.
Figure 7 is an illustration of a flow diagram of a method for determining a position of an electrically conductive, non-ferromagnetic element coupled to a distal end of a basket assembly according to aspects of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention. A tubular structure or tube may have a hollow or solid core.

Alternative apparatus and system features and alternative method steps are presented in example embodiments herein. Each given example embodiment presented herein can be modified to include a feature and/or method step presented with a different example embodiment herein where such feature and/or step is compatible with the given example as understood by a person skilled in the pertinent art as well as where explicitly stated herein. Such modifications and variations are intended to be included within the scope of the claims.

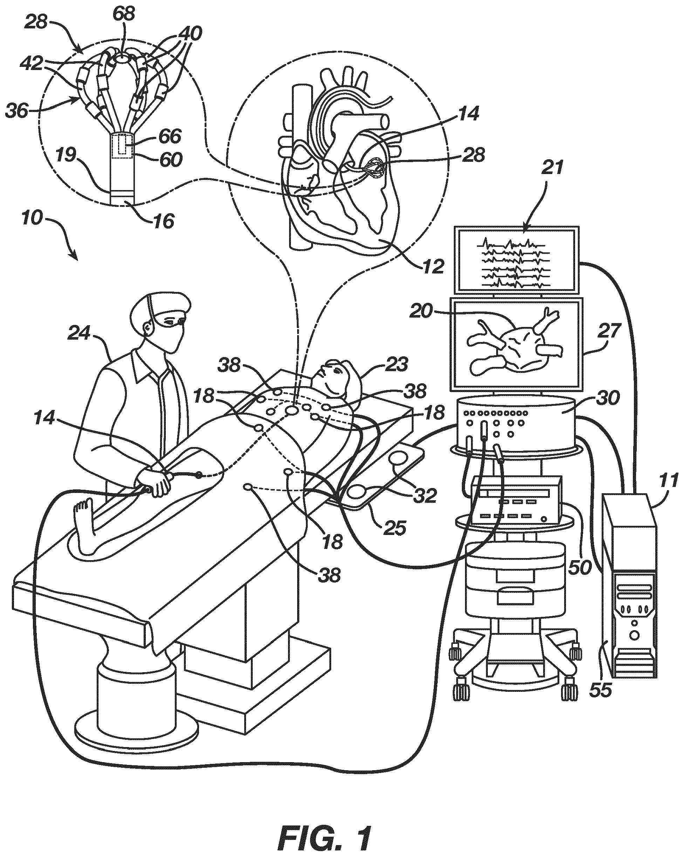

Figure 1 is an illustration showing an examplary catheter-based electrophysiology mapping and ablation system 10. The system 10 includes multiple catheters, which are percutaneously inserted by a physician 24 through the patient's vascular system into a chamber or vascular structure of a heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. The physician 24 brings a basket assembly 36 (end effector) of the distal portion 28 of the catheter 14 into contact with the heart wall for sensing IEGM signals and/or ablation of a target site in the heart 12. The catheter 14 includes a shaft 16 having a proximal portion that can be manipulated (e.g. by a handle) by the physician 24, external to the patient 23 to position a distal portion of the shaft 16 into the heart 12.

The illustrated catheter 14 is an exemplary catheter that includes one and preferably multiple electrodes 40 distributed over a plurality of spines 42 of the basket assembly 36 and configured to sense the IEGM signals and/or apply electrical signals for RF and/or IRE ablation. Catheter 14 may additionally include a position sensor 66 embedded in or near the distal portion 28 (e.g. embedded in a shaft 16 of the catheter 14) for tracking position and orientation of distal portion 28. Optionally and preferably, the position sensor 66 includes a magnetic based position sensor including three magnetic coils for sensing three-dimensional (3D) position and orientation.

A magnetic based position sensor 66 may be operated together with a location pad 25 including a plurality of magnetic coils 32 configured to generate magnetic fields in a predefined working volume. Real time position of the distal portion 28 of the catheter 14 may be tracked based on magnetic fields generated with a location pad 25 and sensed by a magnetic based position sensor 66. Navigation signals can be transmitted from the magnetic based position sensor 66 which can be used by the system 10 to determine a location and orientation of the magnetic based position sensor 66 within the predefined working volume. Details of the magnetic based position sensing technology are described in U.S. Patent Nos. 5,391,199, 5,443,489, 5,558,091, 6,172,499, 6,239,724, 6,332,089, 6,484,118, 6,618,612, 6,690,963, 6,788,967, and 6,892,091 incorporated by reference herein.

The system 10 includes one or more electrode patches 38 positioned for skin contact on the patient 23 to establish location reference for location pad 25 as well as impedance-based tracking of electrodes 40 coupled to the spines 42 and/or an electrode 19 coupled to the shaft 16 of the catheter 14 in the distal portion 28. For impedance-based tracking, electrical current is directed toward electrodes of the distal portion 28 of the catheter 14 and sensed at electrode skin patches 38 so that the location of each catheter electrode can be triangulated via the electrode patches 38. Details of the impedance-based location tracking technology are described in US Patent Nos. 7,536,218, 7,756,576, 7,848,787, 7,869,865, and 8,456,182 incorporated by reference herein.

A recorder 11 displays electrograms 21 captured with body surface electrocardiogram (ECG) electrodes 18 and may also display IEGM signals captured with electrodes 40 of the catheter 14. The recorder 11 may include pacing capability for pacing the heart rhythm and/or may be electrically connected to a standalone pacer.

The system 10 can include an ablation energy generator 50 that is adapted to conduct ablative energy to one or more of electrodes 40 of the basket assembly 36 configured for ablating. Energy produced by the ablation energy generator 50 may include, but is not limited to, RF energy or pulsed-field ablation (PFA) energy, including monopolar or bipolar high-voltage direct current (DC) pulses as may be used to effect IRE, or combinations thereof. In one example, the electrodes 40 and ablation energy generator 50 are configured to provide at least 900 volts (V) between electrodes 40 to ablate tissue with IRE.

A patient interface unit (PIU) 30 is an interface configured to establish electrical communication between catheters, electrophysiological equipment, a power supply, and a workstation 55, and other such computational or medical equipment for controlling operation of system 10. Electrophysiological equipment of the system 10 may include for example, multiple catheters, a location pad 25, body surface ECG electrodes 18, electrode patches 38, an ablation energy generator 50, and a recorder 11. Optionally and preferably, the PIU 30 includes processing capability for implementing real-time computations of location of the catheters and for performing ECG calculations.

The workstation 55 includes memory, processor unit with memory or storage with appropriate operating software loaded therein, and user interface capability. The workstation 55 can be configured to provide multiple functions, optionally including (1) modeling the endocardial anatomy in 3D and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on the display device 27 sites of interest such as places where ablation energy has been applied.

The system 10 can be configured to facilitate image guided surgery (IGS). The IGS system may enable the physician to visually track the location of the catheter 14 within the patient 23, in relation to images of anatomical structures (e.g. heart 12) within the patient 23, in real time.

One commercial product embodying elements of the system 10 is available as the CARTO^{™} 3 System, available from Biosense Webster, Inc., 31A Technology Drive, Irvine, CA 92618.

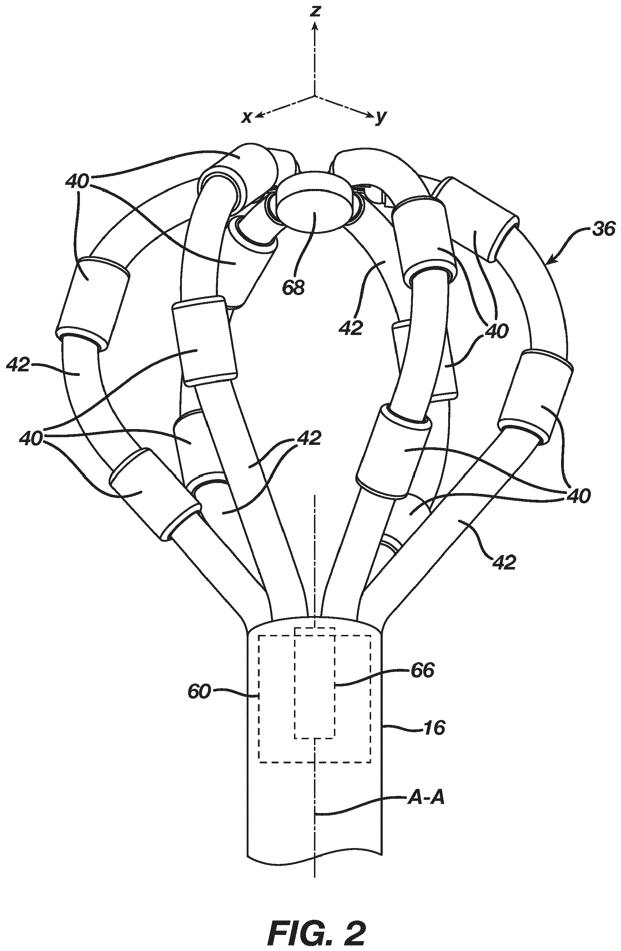

Figure 2 is an illustration of the distal portion 28 of the catheter 14. The shaft 16 is aligned along a longitudinal axis A-A. The spines 42 extend from a distal end of the shaft 16 and provide structure support for the basket assembly 36. The spines 42 are configured to be aligned with the longitudinal axis A-A when the distal portion 28 is delivered through vasculature (e.g. via a sheath) to a target treatment site, and the spines 42 are configured to expand away from the longitudinal axis A-A to form a resilient basket. Each spine 42 can include a resilient strut or frame, formed of a flexible, resilient material, preferably a shape-memory alloy such as nickel-titanium, also known as Nitinol. Each spine 42 can further include an insulating jacket over each strut or frame to electrically isolate electrodes 40 from the underlying strut or frame. The insulating jacket can include one or more lumens through which electrical signal conductors can extend to provide electrical contact to the electrodes 40 to communicate with the PIU 30 (Figure 1).

The location element 68 is coupled to distal ends of the spines 42. The location element 68 is configured to move in relation to the transmitting coil 64 in response to a force on the basket assembly 36. Because the location element 68 does not require an electrical signal conductor (e.g. wire or flex circuit trace) to transmit signals to the PIU 30 (Figure 1), no electrical signal conductors need extend beyond the distal-most electrode 40 of a spine. The basket assembly 36 can therefore be made more symmetrically responsive to external forces applied to basket assembly 36 because there is no electrical signal conductor to the location element 68 affecting resiliency of a spine differently than spines lacking an electrical signal conductor distal of the electrodes. Further, there is possibility of breakage of such an electrical signal conductor due to flexion of the basket assembly because the location element 68 does not require an electrical signal conductor.

The location element 68 can include an electrically conductive, non-ferromagnetic material. The location element 68 can include a diamagnetic or paramagnetic material. The location element 68 is further biocompatible. Aluminum and stainless steel are two examples of electrically conductive, non-ferromagnetic material that has a high electrical conductivity and is a suitable material for the location element 68. Iron, cobalt, and nickel are ferromagnetic, and therefore not suitable. Examples of non-ferromagnetic metals also include gold, silver, copper, titanium, bismuth, lithium, lead, brass, and bronze, but are not necessarily suitable if not biocompatible or lack sufficient electrical conductivity.

The coil assembly 60 is disposed in the shaft 16 near the distal end of the shaft 16. Windings and electrical signal conductors to of the coil assembly 60, in general, can be made larger than inductive coils and electrical signal conductors of the basket assembly 36 because the spines 42 are smaller in diameter than the shaft 16. Further, the shaft 16 experiences less mechanical deformation during a treatment compared to the basket assembly 36 so that inductive coils and electrical signal conductors in the shaft 16 generally experience less mechanical stress and are therefore generally less prone to breakage compared to inductive coils and electrical signal conductors of the shaft.

The catheter 14 can further be altered to include compatible features of other basket catheter designs such as disclosed in U.S. Patent Pub. Nos. 2021/0307815, 2022/0071693, 2022/0071695, 2022/0071696, 2022/0087734, 2022/0087735, 2022/0193370, 2022/0361942, 2023/0015298, 2023/0075838, 2023/0112251, and 2023/0138104, each of which are incorporated herein by reference and attached in the Appendix of priority U.S. Provisional Patent Application No. 63/507,164.

Referring collectively to Figure 1 and Figure 2, the system 10 may further be configured to sense deflection of the basket assembly 36. The coil assembly 60 can transmit a first alternating magnetic field to the location element 68 which induces eddy currents in the location element 68 causing a second magnetic field to be generated which is sensed by the coil assembly 60. The coil assembly 60 can be in electrical communication with a computational module of the system 10 which includes a signal processor having a processor and non-transitory computer readable medium having instructions thereon, that when executed by the processor, causes the processor to determine a position of the location element 68 in relation the coil assembly 60 based at least in part on the signals and/or data from the coil assembly 60. The computational module may further include a signal generator configured to provide an output signal to the coil assembly 60 to generate the first alternating magnetic field. The computational module may be included in the PIU 30, the workstation 55, distributed between the PIU 30 and workstation 55, distributed between elements of the illustrated system 10 and an external computational device or system (e.g. network/cloud computational device or system). Preferably, the PIU 30 includes the signal generator and signal processor.

The signal processor may further be configured to determine a direction and magnitude of a force applied to spines 42 of the basket assembly 36 based at least in part on a comparison of the output signal to the input signals. The signal processor may further be configured to determine a deformed shape of the spines 42 of the basket assembly 36 based at least in part on a comparison of the output signal to the input signals. The system 10 may further include a visualization module configured to graphically render the deformed shape for display on a display device (e.g. display 27). The visualization module may further be configured to graphically render the deformed shape in relation to an anatomical map (e.g. of heart 12). The visualization module may include a processor and non-transitory computer readable medium with instructions thereon, that when executed by the processor, cause the processor to graphically render the deformed shape. The visualization module may be included in the PIU 30, the workstation 55, distributed between the PIU 30 and workstation 55, distributed between elements of the illustrated system 10 and an external computational device or system (e.g. network/cloud computational device or system). Preferably, the PIU 30 includes the visualization module.

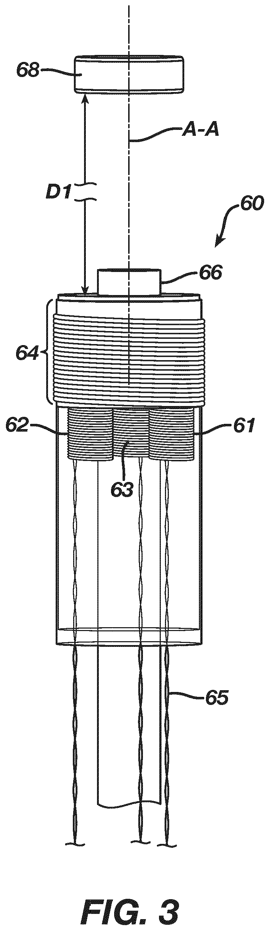

Figure 3 is an illustration of the coil assembly 60 and location element 68 of the catheter 14. The coil assembly 60 includes a transmitting coil 64 and receiving coils 61, 62, 63. The coil assembly 60 may further include a magnetic based position sensor 66 (also referred to herein as a three-axis sensor) disposed within the shaft 16 and configured to output signals indicative of a position and orientation of the three-axis sensor 66 relative to a magnetic field generated externally from the medical probe. At least a portion of the three-axis sensor 66 can be circumscribed by the transmitting coil 64.

The transmitting coil 64 is disposed within the shaft 16, aligned along the longitudinal axis A-A and configured to generate a first alternating magnetic field. The location element 68 is coupled to distal ends of the spines 42. The location element 68 is configured to move in relation to the transmitting coil 64 in response to a force on the basket assembly 36. The location element 68 is configured to generate a second alternating magnetic field based at least in part on the first alternating magnetic field and based in part on a position of the location element 68 in relation to the transmitting coil 64.

The transmitting coil 64 is preferably coaxial with the longitudinal axis A-A. The location element 68 is preferably centered at the longitudinal axis and has a surface that is orthogonal to the longitudinal axis A-A and facing proximally toward the coil assembly 60 when the basket assembly 36 is in the expanded basket configuration in free space (i.e. without an externally applied force to the basket assembly 36). The location element 68 is preferably disk or ring shaped. The transmitting coil 64 and location element 68 are preferably coaxial when the basket assembly 36 is in the expanded basket configuration in free space. The transmitting coil 64 is separated from the location element 68 by a predetermined distance D1 when the basket assembly 36 is in the expanded basket configuration in free space.

The receiving coils 61, 62, 63 are disposed within the shaft 16 and are each configured to output a respective electrical signal based at least in part on a superposition of the first alternating magnetic field and the second alternating magnetic field. The receiving coils 61, 62, 63 can each be aligned along the longitudinal axis A-A. The receiving coils 61, 62, 63 can each be circumscribed by the transmitting coil 64.

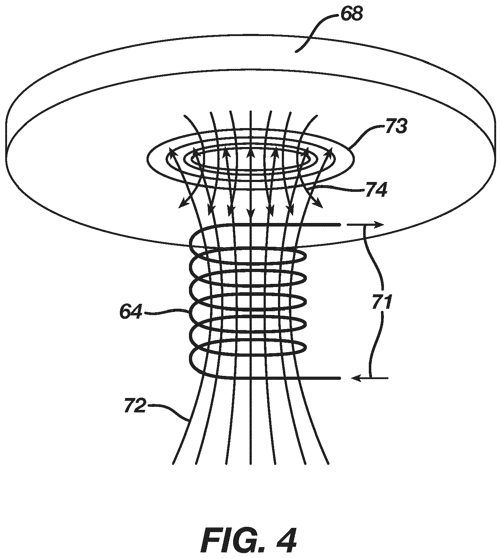

Figure 4 is an illustration of the transmitting coil 64 inducing a first alternating magnetic field 72, which induces eddy currents 73 on the location element 68, thereby inducing a second alternating magnetic field 74. The second alternating magnetic field 74 opposes the first alternating magnetic field 72 so that a receiving coil that is closer, or more aligned with, the second alternating magnetic field 74 experiences a lower magnitude composite field from the first and second alternating magnetic fields compared to a receiving coil that is further from, or less aligned with, the second alternating magnetic field 74.

Note that the first and second magnetic field can be distinguished from the magnetic fields generated by the magnetic coils 32 external to the patient 23 that are configured to generate magnetic fields in the predefined working volume and sensed by the magnetic based position sensor 66. For instance, magnetic fields generated by magnetic coils 32 external to the patient 23 can have frequency that is distinct from frequency of the first and second alternating magnetic fields. The first and second alternating magnetic fields are not necessarily distinguishable from each other because the second magnetic field is generated based on the first alternating magnetic field.

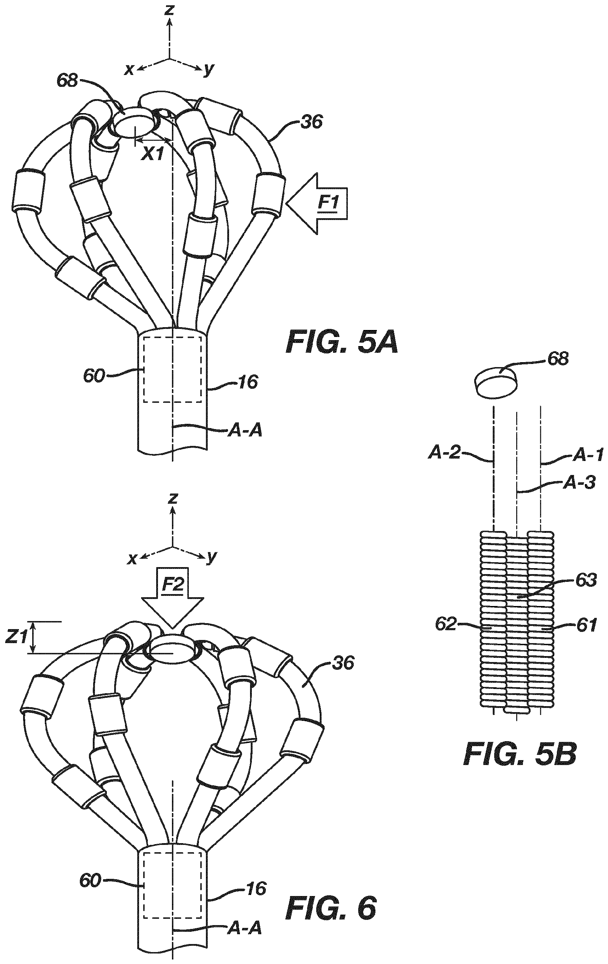

Figure 5A is an illustration of deformation of the basket assembly 36 in response to a force F1 in an x-direction. A center of the location element 68 is shifted by a distance X1 in the x-direction from the longitudinal axis A-A. In this example, the transmitting coil 64 remains separated from the location element 68 by the predetermined distance D1, or approximately the predetermined distance D1 (Figure 3).

Figure 5B is an illustration of the location element 68 and receiving coils 61, 62, 63 of the catheter 14 of Figure 5A. Each receiving coil 61, 62, 63 has a respective axis A-1, A-2, A-3 that is aligned along the longitudinal axis A-A and separated from the longitudinal axis A-A. The axes A-1, A-2, A-3 of the receiving coils 61, 62, 63 are preferably equally distributed around the longitudinal axis A-A. In the illustration, movement of the location element 68 in the x-direction moves the location element 68 in alignment with an axis A-2 of one of the receiving coils 62 while moving the location element 68 away from the axes A-1, A-3 of the other receiving coils 61, 63. The second alternating magnetic field 74 (Figure 4) therefore interacts more with the coaxial receiving coil 62 than the other receiving coils 61, 63. The second alternating magnetic field 74 opposes the first alternating magnetic field so that the aligned receiving coil 62 experiences a lower magnitude composite field from the first and second alternating magnetic fields compared to the other receiving coils 61, 63. The system 10 can therefore be configured to detect deflection of the basket assembly from the longitudinal axis A-A based on difference in magnitude of signals between the three receiving coils 61, 62, 63.

Figure 6 is an illustration of deformation of the basket assembly 36 in response to a force F2 in a z-direction. The location element 68 is moved a distance Z1 proximally due to the force F2. The distance between the location element 68 and the coil assembly 60 can therefore been reduced from the predetermined distance D1 (Figure 3) by the distance Z1. The second alternating magnetic field 74 (Figure 4) is received more strongly by the receiving coils 61, 62, 63 of the coil assembly 60 because of this movement, thereby reducing the magnitude of the superposition of the first and second alternating magnetic fields. Because the location element 68 remains aligned with the longitudinal axis A-A, the reduction in the magnitude of the superposition of the first and second alternating magnetic fields is experienced equally by the receiving coils 61, 62, 63. The system 10 can therefore be configured to detect compression of the basket assembly along the longitudinal axis A-A based on a change in magnitude of signals on the three receiving coils 61, 62, 63 from a predetermined magnitude measured when the basket assembly 36 is not deformed by a force.

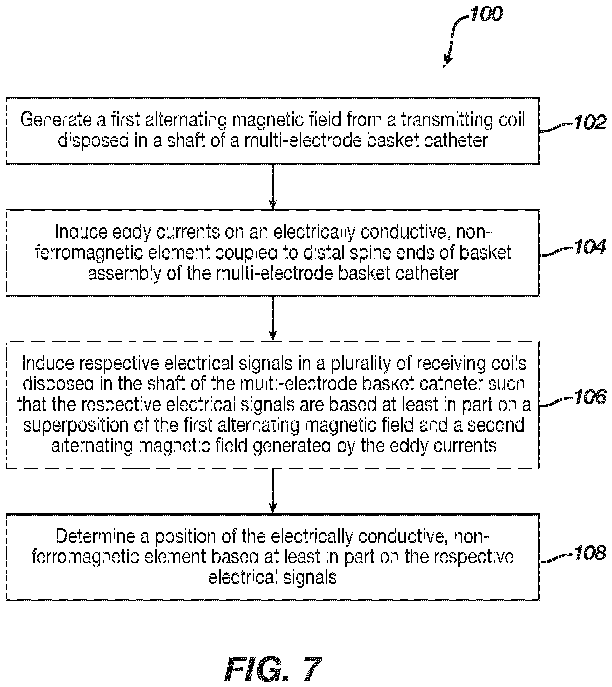

Figure 7 is an illustration of a flow diagram of a method 100 for determining a position of an electrically conductive, non-ferromagnetic element coupled to a distal end of a basket assembly. The electrically conductive, non-ferromagnetic element can be configured similar to location element 68, an alternative thereto, or variation thereof as understood by a person skilled in the pertinent art. The method 100 can be included as instructions on non-transitory computer readable memory that can be coupled to one or more processors of system 10 (or alternative system as understood by a person skilled in the pertinent art) so that when the instructions of the method 100 are executed by the one or more processors, the system 10 is configured to perform the steps of the method 100.

At step 102, a first alternating magnetic field can be generated from a transmitting coil disposed in a shaft of a multi-electrode basket catheter. The multi-electrode basket catheter can be configured similar to catheter 14 illustrated herein, variations thereof, and alternatives thereto as understood by a person skilled in the pertinent art. The transmitting coil can be configured similar to transmitting coil 64 illustrated herein, variations thereof, and alternatives thereto as understood by a person skilled in the pertinent art. The first alternating magnetic field can be generated by the PIU 30, other component of the system 10 illustrated herein, variations thereof, and alternatives thereto as understood by a person skilled in the pertinent art.

At step 104 eddy currents can be induced on the electrically conductive, non-ferromagnetic element, which is coupled to distal spine ends of a basket assembly of the multi-electrode basket catheter.

At step 106, respective electrical signals can be induced in a plurality of receiving coils disposed in the shaft of the multi-electrode basket catheter such that the respective electrical signals are based at least in part on a superposition of the first alternating magnetic field and a second alternating magnetic field generated by the eddy currents.

At step 108, a position of the electrically conductive, non-ferromagnetic element can be determined based at least in part on the respective electrical signals.

The following clauses list non-limiting embodiments of the disclosure:
Clause 1. A medical probe comprising: a shaft extending along a longitudinal axis; a plurality of spines extending from a distal end of the shaft and configured to expand away from the longitudinal axis to form a resilient basket; one or more electrodes coupled to each spine of the plurality of spines; a transmitting coil disposed within the shaft, aligned along the longitudinal axis and configured to generate a first alternating magnetic field; a location element coupled to distal ends of the spines, configured to move in relation to the transmitting coil in response to a force on the resilient basket, and configured to generate a second alternating magnetic field based at least in part on the first alternating magnetic field and based in part on a position of the location element in relation to the transmitting coil; and a plurality of receiving coils disposed within the shaft and configured to output a respective electrical signal based at least in part on a superposition of the first alternating magnetic field and the second alternating magnetic field.
Clause 2. The medical probe of clause 1, the location element comprising an electrically conductive, non-ferromagnetic material.
Clause 3. The medical probe of clause 1 or 2, the location element comprising aluminum.
Clause 4. The medical probe of any one of clauses 1-3, the transmitting coil and the location element being configured such that the first alternating magnetic field induces eddy currents in the location element and the eddy currents generate the second alternating magnetic field.
Clause 5. The medical probe of any one of clauses 1-4, the plurality of receiving coils each being aligned along the longitudinal axis.
Clause 6. The medical probe of any one of clauses 1-5, the plurality of receiving coils comprising three receiving coils disposed symmetrically about the longitudinal axis.
Clause 7. The medical probe of any one of clauses 1-6, wherein at least a portion of the plurality of receiving coils is circumscribed by the transmitting coil.
Clause 8. The medical probe of any one of clauses 1-7, further comprising: a three-axis sensor disposed within the shaft and configured to output signals indicative of a position and orientation of the three-axis sensor relative to a magnetic field generated externally from the medical probe.
Clause 9. The medical probe of clause 8, wherein at least a portion of the three-axis sensor is circumscribed by the transmitting coil.
Clause 10. The medical probe of any one of clauses 1-9, the shaft comprising a proximal portion and a distal portion and being configured to be manipulated at the proximal portion to position the distal portion into a heart of a patient.
Clause 11. The medical probe of any one of clauses 1-10, the one or more electrodes configured to sense intracardiac electrogram signals.
Clause 12. The medical probe of any one of clauses 1-11, the one or more electrodes configured to provide radio frequency signals to ablate tissue.
Clause 13. The medical probe of any one of clauses 1-12, the one or more electrodes configured provide at least 900 V between electrodes to ablate tissue with irreversible electroporation.
Clause 14. A medical system comprising: a signal generator configured to provide an output signal to a transmitting coil disposed in a shaft of a medical probe; and a signal processor configured to: receive a plurality of input signals from a plurality of receiving coils disposed in the shaft of a multi-electrode basket catheter, and determine a position of an electrically conductive, non-ferromagnetic element coupled to distal ends of spines of the multi-electrode basket catheter based at least in part on a comparison of the output signal to the plurality of input signals.
Clause 15. The medical system of clause 14, the multi-electrode basket catheter being configured such that the output signal generates a first alternating magnetic field by the transmitting coil, the first alternating magnetic field induces eddy currents in the electrically conductive, non-ferromagnetic element, the eddy currents generate a second alternating magnetic field, and a superposition of the first alternating magnetic field and the second alternating magnetic field affects the plurality of input signals.
Clause 16. The medical system of clause 14 or 15, the signal processor further being configured to: determine a direction and magnitude of a force applied to spines of the multi-electrode basket catheter based at least in part on a comparison of the output signal to the plurality of input signals.
Clause 17. The medical system of any one of clauses 14-16, the signal processor further being configured to: determine a deformed shape of the spines of the multi-electrode basket catheter based at least in part on a comparison of the output signal to the plurality of input signals.
Clause 18. The medical system of clause 17, further comprising: a visualization module configured to graphically render the deformed shape for display on a display device.
Clause 19. The medical system of clause 18, wherein the visualization module is further configured to graphically render the deformed shape in relation to an anatomical map.
Clause 20. The medical system of any one of clauses 14-19, further comprising: an intracardiac electrogram (IEGM) sensor configured to receive IEGM signals from electrodes of the multi-electrode basket catheter.
Clause 21. The medical system of any one of clauses 14-20, further comprising: a radio frequency (RF) ablation energy generator configured to provide RF signals to electrodes of the multi-electrode basket catheter to perform RF ablation of tissue.
Clause 22. The medical system of any one of clauses 14-21, further comprising: a pulse-field ablation (PFA) energy generator configured to provide pulsed signals to electrodes of the multi-electrode basket catheter to perform ablation by irreversible electroporation.
Clause 23. The medical system of any one of clauses 14-22, further comprising: a magnetic field generator configured to generate a magnetic field in a pre-defined working volume external; and a magnetic tracking signal processor configured to: receive navigation signals from a three-axis sensor disposed in the shaft of the multi-electrode basket catheter, and determine a location and an orientation of the three-axis sensor within the pre-defined working volume based at least in part on the navigation signals.
Clause 24. A method comprising: generating a first alternating magnetic field from a transmitting coil disposed in a shaft of a multi-electrode basket catheter; inducing eddy currents on an electrically conductive, non-ferromagnetic element coupled to distal spine ends of a basket assembly of the multi-electrode basket catheter; inducing respective electrical signals in a plurality of receiving coils disposed in the shaft of the multi-electrode basket catheter such that the respective electrical signals are based at least in part on a superposition of the first alternating magnetic field and a second alternating magnetic field generated by the eddy currents; and determining a position of the electrically conductive, non-ferromagnetic element based at least in part on the respective electrical signals.
Clause 25. The method of clause 24, further comprising: determining a direction and magnitude of a force applied to basket assembly based at least in part on the respective electrical signals.
Clause 26. The method of clause 24 or 25, further comprising: determining a deformed shape of the basket assembly based at least in part on the respective electrical signals.
Clause 27. The method of clause 26, further comprising: graphically rendering the deformed shape.
Clause 28. The method of clause 26, further comprising: graphically rendering the deformed shape in relation to an anatomical map.
Clause 29. The method of any one of clauses 24-28, further comprising: receiving intracardiac electrogram signals from electrodes of the multi-electrode basket catheter.
Clause 30. The method of any one of clauses 24-29, further comprising: generating radio frequency ablation energy at electrodes of the multi-electrode basket catheter.
Clause 31. The method of any one of clauses 24-29, further comprising: generating pulse-field ablation energy at electrodes of the multi-electrode basket catheter.
Clause 32. The method of any one of clauses 24-31, further comprising: determining a location and orientation of a three-axis sensor disposed within the shaft of the multi-electrode basket catheter.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A medical probe comprising:
a shaft extending along a longitudinal axis;
a plurality of spines extending from a distal end of the shaft and configured to expand away from the longitudinal axis to form a resilient basket;
one or more electrodes coupled to each spine of the plurality of spines;
a transmitting coil disposed within the shaft, aligned along the longitudinal axis and configured to generate a first alternating magnetic field;
a location element coupled to distal ends of the spines, configured to move in relation to the transmitting coil in response to a force on the resilient basket, and configured to generate a second alternating magnetic field based at least in part on the first alternating magnetic field and based in part on a position of the location element in relation to the transmitting coil; and
a plurality of receiving coils disposed within the shaft and configured to output a respective electrical signal based at least in part on a superposition of the first alternating magnetic field and the second alternating magnetic field.

2. The medical probe of claim 1, the location element comprising an electrically conductive, non-ferromagnetic material.

3. The medical probe of claim 1 or claim 2, the transmitting coil and the location element being configured such that the first alternating magnetic field induces eddy currents in the location element and the eddy currents generate the second alternating magnetic field.

4. The medical probe of any preceding claim, the plurality of receiving coils each being aligned along the longitudinal axis.

5. The medical probe of any preceding claim, the plurality of receiving coils comprising three receiving coils disposed symmetrically about the longitudinal axis, wherein at least a portion of the plurality of receiving coils is circumscribed by the transmitting coil.

6. The medical probe of any preceding claim, further comprising:
a three-axis sensor disposed within the shaft and configured to output signals indicative of a position and orientation of the three-axis sensor relative to a magnetic field generated externally from the medical probe.

7. The medical probe of any preceding claim, the one or more electrodes configured to sense intracardiac electrogram signals, provide radio frequency signals to ablate tissue, and/or provide at least 900 V between electrodes to ablate tissue with irreversible electroporation.

8. A medical system comprising:
a signal generator configured to provide an output signal to a transmitting coil disposed in a shaft of a medical probe; and
a signal processor configured to:
receive a plurality of input signals from a plurality of receiving coils disposed in the shaft of a multi-electrode basket catheter, and
determine a position of an electrically conductive, non-ferromagnetic element coupled to distal ends of spines of the multi-electrode basket catheter based at least in part on a comparison of the output signal to the plurality of input signals.

9. The medical system of claim 8, the multi-electrode basket catheter being configured such that the output signal generates a first alternating magnetic field by the transmitting coil, the first alternating magnetic field induces eddy currents in the electrically conductive, non-ferromagnetic element, the eddy currents generate a second alternating magnetic field, and a superposition of the first alternating magnetic field and the second alternating magnetic field affects the plurality of input signals.

10. The medical system of claim 8 or claim 9, the signal processor further being configured to:
determine a direction and magnitude of a force applied to spines of the multi-electrode basket catheter based at least in part on a comparison of the output signal to the plurality of input signals.

11. The medical system of any of claims 8 to 10, the signal processor further being configured to:
determine a deformed shape of the spines of the multi-electrode basket catheter based at least in part on a comparison of the output signal to the plurality of input signals.

12. The medical system of claim 11, further comprising:
a visualization module configured to graphically render the deformed shape for display on a display device and graphically render the deformed shape in relation to an anatomical map.

13. The medical system of any of claims 8 to 12, further comprising:
an intracardiac electrogram (IEGM) sensor configured to receive IEGM signals from electrodes of the multi-electrode basket catheter.

14. The medical system of any of claims 8 to 13, further comprising:
an ablation energy generator configured to provide RF signals and/or pulsed signals to electrodes of the multi-electrode basket catheter to perform RF ablation and/or irreversible electroporation of tissue.
